# EUROPÄISCHE PATENTSCHRIFT

(11) **EP 0 470 437 B1**
(45) Veröffentlichungstag und Bekanntmachung des Hinweises auf die Patenterteilung: **29.11.1995**
(21) Anmeldenummer: 91112377.6
(22) Anmeldetag: 24.07.1991
(51) Int. Cl.: A61K 9/127

(54) **Wässriges Liposomensystem**
Aqueous liposome system
Système aqueux de liposomes

(30) Priorität: 06.08.1990 DE 4024886; 19.03.1991 DE 4108902; 10.07.1991 DE 4122744
(43) Veröffentlichungstag der Anmeldung: 12.02.1992
(73) Patentinhaber: A. Nattermann & Cie. GmbH, D-50829 Köln (DE)
(72) Erfinder: Hager, Jörg, W-5000 Köln 30 (DE); Dürr, Manfred Dr., W-5010 Bergheim-Glessen (DE); Lünebach, Ernst Dr., W-5024 Erftstadt-Lechenich (DE)
(74) Vertreter: Döring, Wolfgang, Dr.-Ing. Patentanwälte Hauck, Graalfs, Wehnert, Döring, Siemons

(56) Entgegenhaltungen:
- EP-A- 0 274 174
- EP-A- 0 315 467
- EP-A- 0 331 635
- DE-A- 3 301 951
- US-A- 4 348 384
- US-A- 4 744 989
- Can. J. Biochem. Physiol. Vol 37 (1959) Seiten 953-959

## Beschreibung

Die vorliegende Erfindung betrifft ein wäßriges Liposomensystem nach dem Oberbegriff des Patentanspruchs 1 sowie ein Verfahren zur Herstellung eines derartigen Liposomensystems.

Phospholipidische Liposomensysteme sind für verschiedene Anwendungen bekannt. So werden diese Systeme beispielsweise im kosmetischen Bereich oder für die Herstellung von pharmazeutischen Produkten eingesetzt. Dabei sind die jeweiligen Wirkstoffe in den als Liposomen bezeichneten Kugeln (Vesikel) eingekapselt, wobei diese Liposome vorzugsweise in ihrem Inneren eine wäßrige Phase enthalten, in der der entsprechende Wirkstoff dann entsprechend gelöst, dispergiert oder emulgiert ist. Nach außen sind die Liposomen durch eine Lipiddoppelmembran begrenzt.

So beschreiben beispielsweise die EP 03 09 519 A1 und die EP 03 15 467 A1 Liposomensysteme, die den Wirkstoff Pentamidin einkapseln und die als entsprechende pharmazeutische Produkte verwendet werden.

Desweiteren sind aus der US 4,348,304 eine liposomale pharmazeutische Formulierung zur Behandlung von Hämophilie, aus der EP 0 331 635 A2 ein Krebsbehandlungsmittel und aus der EP 0 274 174 A1 ein Leberbehandlungsmittel bekannt, wobei die zuvor genannten bekannten pharmazeutischen Zubereitungen alle ein Phospholipid sowie einen negativen phospholipidischen Ladungsträger enthalten. Das Massenverhältnis von Phospholipid zu dem negativen phospholipidischen Ladungsträger liegt jedoch bei den bekannten Zubereitungen weit oberhalb eines Bereiches von 50:1 bis 400:1.

Ein wäßriges Liposomensystem mit den Merkmalen des Oberbegriffs des Patentanspruchs 1 ist aus der US 4,744,989 A1 zu entnehmen. Hierbei wird ein wasserlösliches Carriermaterial, das mit einer liposomalen Schicht beschichtet ist, dann durch Zugabe von Wasser in ein wäßriges Liposomensystem umgewandelt, wobei dieses wäßrige Liposomensystem zwangsläufig immer in seinem Inneren eine Lösung des wasserlöslichen Carriers enthält. Mit anderen Worten besteht somit das aus der US 4,744,989 A1 bekannte wäßrige Liposomensystem zwangsläufig immer aus dem Phosphatidylcholin, dem mindestens einen phospholipidischen Ladungsträger, Wasser und dem im Inneren der Liposomen angeordneten wasserlöslichen Carrier, wobei die Reinheit des eingesetzten Phosphatidylcholins in der US 4,744,989 A1 nicht quantifiziert ist.

Die bekannten Liposomensysteme weisen häufig den Nachteil auf, daß sie alle die Tendenz besitzen, schon nach kurzer Zeit einen unerwünschten Bodensatz auszubilden, so daß sie trüb werden und somit nicht ohne weiteres auf Fremdpartikel zu kontrollieren sind.

Der vorliegenden Erfindung liegt die Aufgabe zugrunde, ein wäßriges phospholipidisches Liposomensystem zur Verfügung zu stellen, das eine besonders hohe Stabilität und eine besonders hohe Transparenz besitzt.

Diese Aufgabe wird erfindungsgemäß durch ein wäßriges Liposomensystem mit den kennzeichnenden Merkmalen des Patentanspruchs 1 gelöst.

Das erfindungsgemäße wäßrige Liposomensystem besteht aus einem hochreinem Phosphatidylcholin, das weniger als 10 Gew.% Verunreinigungen aufweist, mindestens einem phospholipidischen Ladungsträger sowie ggf. mindestens einer pharmazeutisch wirksamen Substanz und ggf. einen nicht toxischen organischen Lösungsmittel sowie Wasser.

Das erfindungsgemäße Liposomensystem weist eine Reihe von Vorteilen auf. So konnte festgestellt werden, daß das erfindungsgemäße Liposomensystem auch bei einer extrem langen Lagerzeit von mehreren Monaten bis Jahren keine Tendenz zeigte, einen Bodensatz oder Ablagerungen an den Gefäßwandungen auszubilden. Des weiteren besitzt das erfindungsgemäße Liposomensystem eine hohe Transparenz und ist nicht, wie bei den bekannten Liposomensystemen, milchig trüb. Dies wiederum führt dazu, daß bei dem erfindungsgemäßen Liposomensystem ohne Schwierigkeiten eine Prüfung auf Fremdpartikeln durchführbar ist, da es hierfür lediglich erforderlich ist, die entsprechende Liposomendispersionen im Durchsichtverfahren zu überprüfen. Auch ist es steril filtrierbar, so daß sich das erfindungsgemäße Liposomensystem insbesondere für eine pharmazeutische, kosmetische oder diätetisch Anwendung eignet.

Bezüglich der Massenverhältnisse des hochreinen Phosphatidylcholins zu dem mindestens einen negativen phospholipidischen Ladungsträger ist festzuhalten, daß dieses Massenverhältnis zwischen 50:1 bis 400:1, vorzugsweise zwischen 100:1 bis 200:1, variiert. Hierbei konnte festgestellt werden, daß bereits kleine Mengen des negativen Ladungsträgers ausreichen, um dem hieraus erstellten phospholipidische Liposomensystem die vorstehend beschriebene Stabilität bei einer Lagerung sowie eine hohe Transparenz zu verleihen. Hinzu kommt noch, daß das erfindungsgemäße Liposomensystem mit wesentlich geringerem Aufwand und somit in etwa der halben Zeit durch Hochdruckspalthomogenisation oder Ultraschall auf einen gewünschten mittleren Partikeldurchmesser, der zwischen 50 nm und 180 nm, vorzugsweise zwischen 70 nm und 130 nm, liegt, homogen zerkleinert werden kann. Auch läßt sich das erfindungsgemäße Liposomensystem ohne Problem steril filtrieren, wobei hierfür vorzugsweise 0,2 »m Filter eingesetzt werden.

Die zuvor beschriebenen vorteilhaften Wirkungen des erfindungsgemäßen Liposomensystems werden darauf zurückgeführt, daß es offensichtlich aufgrund der Anwesenheit des hochreinen Phosphatidylcholins und des negativen phospholipidischen Ladungsträgers zu einem synergistischen Effekt kommt.

Besonders gute Ergebnisse bezüglich der zuvor aufgeführten Vorteile weist eine Ausführungsform des erfindungsgemäßen Liposomensystems auf, das als phospholipidischen Ladungsträger mindestens ein Salz, vorzugsweise ein Natrium- und/oder ein Ammoniumsalz, von Phosphatidylglycerol und/oder dessen Derivaten enthält. Hierbei handelt es sich vorzugsweise um das entsprechende Salz von Dimyristoylphosphatidylglycerol und/oder Dipalmitoylphosphatidylglycerol.

Grundsätzlich kann man bei dem erfindungsgemäßen Liposomensystem das Phosphatidylglycerol, das bei den zuvor beschriebenen Ausführungsformen in Form eines entsprechenden Salzes vorliegt und somit den bevorzugten negativen phospholipidischen Ladungsträger bildet, aus jeder natürlichen Substanz, wie beispielsweise aus Ölsaaten, Raps, Sonnenblumen u. dgl., isolieren und dementsprechend ggf. nach einer Reinigung einsetzen. Besonders geeignet ist es jedoch, wenn man die zuvor genannten Salze des Phosphatidylglycerols bzw. der entsprechenden Derivate davon aus Sojabohnen isoliert, so daß vorzugsweise bei dem erfindungsgemäßen Liposomensystem als negativer Ladungsträger ein Soja-Phosphatidylglycerol-Alkalisalz, insbesondere Natrium- oder Kaliumsalz, bzw. ein Soja-Phosphatidylglycerolderivat-Alkalisalz, vorzugsweise Natrium- oder Kaliumsalz, eingesetzt wird.

Bezüglich der Phospholipidkonzentration bei dem erfindungsgemäßßen Liposomensystem ist festzuhalten, daß diese zwischen 0,5 Gew.% und 20 Gew.% variiert.

Wie bereits eingangs beschrieben, kann auch das erfindungsgemäße Liposomensystem sowohl für pharmazeutische als auch für kosmetische Zwecke eingesetzt werden.

Wird das erfindungsgemäße Liposomensystem für pharmazeutische Zwecke verwendet, so bestehen zwei Möglichkeiten.

Hierbei sieht die erste Möglichkeit vor, daß das erfindungsgemäße Liposomensystem in Form eines Leer-Liposomensystems verwendet wird, d.h. das Liposomensystem als solches weist bereits eine pharmazeutische Wirksamkeit auf. Hier konnte festgestellt werden, daß ein derartiges Leer-Liposomensystem hervorragend zur Behandlung von Atherosklerose, erhöhten Blutfettwerten sowie Hepatopathien jeder Genese einsetzbar ist, wobei ein derartiges System vorzugsweise neben Wasser und ggf. Alkohol zwischen 5 Gew.% und 15 Gew.% einer Mischung von hochreinem Phosphatidylcholin und negativem Ladungsträger in dem zuvor genannten Massenverhältnis enthält. Insbesondere wird ein derartiges pharmazeutisches Produkt dann bei seiner Anwendung injiziert.

Die zweite Möglichkeit sieht vor, daß in das erfindungsgemäße Liposomensystem ein Wirkstoff eingekapselt wird. Hier hat sich gezeigt, daß ein derartig eingekapselter Wirkstoff im Vergleich zu der bekannten Aufmachungsform eine verbesserte therapeutische Wirkung, besitzt, ohne daß hierdurch das Behandlungsziel beeinträchtigt wird. Dieser Effekt wird darauf zurückgeführt, daß die in dem Liposomensystem eingekapselten Wirkstoffe besonders gleichmäßig über einen längeren Zeitraum während der therapeutischen Anwendung abgegeben werden, so daß auch unerwünschte Nebenwirkungen nicht auftreten oder zumindestens erheblich reduziert sind.

Die Auswahl des jeweiligen Wirkstoffes richtet sich dann nach dem jeweiligen Anwendungsgebiet. So können beispielsweise in dem erfindungsgemäßen Liposomensystem Pentamidin, Pentamidin-Salze, insbesondere Pentamidin-Isethionat, und/oder Pentamidin-Derivate gelöst und/oder eingekapselt werden, so daß ein derartiges pharmazeutisches Produkt vorzugsweise zur parenteralen und insbesondere zur pulmonalen Behandlung von Pneumocystis-carinii-Pneumonie, der afrikanischen Schlafkrankheit oder von Kala-Azar eingesetzt wird.

Besonders geeignet ist es jedoch, wenn man den zuvor genannten Wirkstoff nicht von Anfang an bei der Herstellung des Liposomensystems einsetzt, sondern den Wirkstoff erst unmittelbar vor der Anwendung zugibt. Dies kann dadurch geschehen, daß man ein wäßriges Liposomensystem mit dem Wirkstoff als Trockensubstanz vermischt oder ein getrocknetes Liposomensystem zunächst in Wasser dispergiert und dann die Vermischung mit dem Wirkstoff herbeiführt. Ein derartig hergestelltes pharmazeutisches Produkt weist dann eine hohe Transparenz auf. In bestimmten Fällen werden ähnliche Effekte erzielt, indem Leer-Liposomen-Präparationen mit dem Wirkstoff kombiniert werden, ohne daß dabei der Wirkstoff in den Liposomen eingekapselt wird.

Weist hingegen das erfindungsgemäße Liposomensystem als Wirkstoff Doxorubicin x HCl auf, so kann es als entsprechendes pharmazeutisches Produkt zur Behandlung von Krebserkrankungen eingesetzt werden.

Soll hingegen das erfindungsgemäße Liposomensystem zur Behandlung von Viruserkrankungen, insbesondere Viruserkrankungen der Haut, verwendet werden, so bietet es sich hier an, einen entsprechenden viruciden Wirkstoff, vorzugsweise Rosmarinsäure oder Dextransulfat, einzukapseln.

Weiterhin können in dem erfindungsgemäßen Liposomensystem auch die entsprechenden bekannten Wirkstoffe zur Behandlung von Krebs, Aids, Leber- oder Viruserkrankungen eingekapselt oder angelagert werden.

Die vorliegende Erfindung betrifft ferner ein Verfahren zur Herstellung des zuvor beschriebenen Liposomensystems.

Das erfindungsgemäße Verfahren zur Herstellung des erfindungsgemäßen Liposomensystems basiert auf der Grunderkenntnis, daß man zunächst das zuvor beschriebene hochreine Phosphatidylcholin zusammen mit dem phospholipidischen Ladungsträger, insbesondere dem Soja-Phosphatidylglycerol-Natriumsalz, in dem vorstehend genannten Massenverhältnis in einem organischen Lösungsmittel löst oder dispergiert. Anschließend engt man die Lösung bzw. Dispersion ein und gibt hiernach eine entsprechende Menge Wasser zu, um so das entsprechende Liposomensystem auszubilden.

Vorzugsweise verwendet man bei dem zuvor beschriebenen erfindungsgemäßen Verfahren als Lösungsmittel Ethanol, Propanol-1 und/oder Propanol-2.

Abhängig von dem jeweils eingesetzten nicht toxischen organischen Lösungsmittel und seiner Mischbarkeit bzw. Verträglichkeit mit Wasser engt man die anfangs hergestellte Lösung bzw. Dispersion auf unterschiedliche Restvolumina ein. Werden z.B. als nicht toxische organische Lösungsmittel die zuvor genannten Alkohole eingesetzt, so bietet es sich hier an, die entsprechende Lösung des Phospholipids mit dem negativen phospholipidischen Ladungsträger auf ein Restvolumen zwischen 3 Vol.% und 30 Vol.%, vorzugsweise 5 Vol.% bis 10 Vol.%, einzuengen. Bei solchen organischen Lösungsmitteln, die mit Wasser nicht mischbar sind, empfiehlt es sich, bis zur Trockenen einzuengen.

Um bei dem erfindungsgemäßen Verfahren ein Liposomensystem herzustellen, das sich durch besonders gleichmäßige, gezielt eingestellte mittlere Liposomendurchmesser auszeichnet, bietet es sich an, nach der Zugabe des Wassers das hierbei entstehende Liposomensystem einer Hochdruckspalthomogenisation oder einer Ultraschallbehandlung zu unterwerfen. Vorzugsweise werden diese Behandlungen solange durchgeführt, bis die dabei gebildeten Liposome einen mittleren Durchmesser zwischen 50 nm und 180 nm aufweisen.

Zusätzlich hierzu kann man hiernach noch das so behandelte Liposomensystem über einen 0,2 »m Filter steril filtrieren.

Die so hergestellten Liposomensysteme können dann entweder direkt anwendungsfertig in entsprechende Ampullen abgefüllt werden oder nach Zugabe geeigneter Hilfsstoffe, insbesondere Kohlehydrate, schonend getrocknet, insbesondere gefriergetrocknet, werden, so daß ein pulverartiges Liposomensystem entsteht, das durch Zugabe einer geeigneten Menge Wasser wieder die dann anwendungsbereiten erwünschten Vesikel ausbildet, ohne daß hierbei ein aufwendigen Rühren oder sonstiges Vermischen erforderlich ist.

Um die Ausführungsformen des erfindungsgemäßen Liposomensystems, die eine der zuvor genannten Wirkstoffe aufweisen, herzustellen, bestehen wiederum zwei Möglichkeiten.

So sieht die erst Möglichkeit vor, daß man den Wirkstoff zusammen mit dem hochreinen Phosphatidylcholin und dem phospholipidischen Ladungsträger direkt zu Beginn des erfindungsgemäßen Verfahrens in das organische Lösungsmittel zugibt. Eine Abwandlung dieser Verfahrensvariante sieht vor, daß man das eingesetzte hochreine Phosphatidylcholin mit dem in einem nicht wäßrigen Lösungsmittel gelösten, dispergierten bzw. emulgierten Wirkstoff belädt, und dann nach schonender Trocknung das so beladene Phosphatidylcholin zusammen mit dem phospholipidischen Ladungsträger in einem organischen Lösungsmittel, das ggf. von dem ersten Lösungsmittel unterschiedlich ist, löst. Anschließend wird das organische Lösungsmittel, wie vorstehend beschrieben, eingeengt und hiernach das Wasser unter Ausbildung des Wirkstoff-Liposomensystem zugegeben, wobei der Wirkstoff dann eingekapselt sein kann. Diese Verfahrensweise bietet sich insbesondere für solche Fälle an, bei denen der Wirkstoff bezüglich seiner Lagerung stabil ist.

Die zweite Möglichkeit, die insbesondere dann bevorzugt wird, wenn der Wirkstoff nicht in dem zuerst eingesetzten organischen Lösungsmittel, sondern in Wasser besser löslich ist, sieht vor, daß man zunächst in der vorstehend beschriebenen Weise das wäßrige Liposomensystem herstellt, wobei man dann zusammen mit dem Wasser den Wirkstoff zugibt.

Eine Abwandlung der zuvor beschriebenen Verfahrensweise, die insbesondere dann angewendet wird, wenn der Wirkstoff nur eine begrenzte Haltbarkeit besitzt, geht von einem pulverförmigen getrockneten Liposomensystem aus. Hierbei wird bei der Redispersierung zusammen mit dem dabei eingesetzten Wasser der Wirkstoff zugesetzt, so daß somit erst unmittelbar vor der Anwendung eines derartigen Produktes der Wirkstoff mit dem Liposomensystem in Kontakt kommt.

Um unerwünschte Nebenwirkungen auszuschließen, wird das erfindungsgemäße Verfahren vorzugsweise unter Schutzgas (Inertgas) durchgeführt.

Vorteilhafte Weiterbildungen des erfindungsgemäßen Verfahrens sind in den Unteransprüchen angegeben.

Das erfindungsgemäße Verfahren wird nachfolgend anhand von Ausführungsbeispielen näher erläutert.

### Beispiel 1

### Herstellung eines Leer-Liposomensystems

99,5 g Phosphatidylcholin hochrein, d.h. weniger als 10 Gew.% Verunreinigungen, und 0,5 g Soja-Phosphatidylglycerol-Natriumsalz (PG) werden in 500 ml Ethanol DAB 9 gelöst und anschließend unter Vakuum zur Trockne gebracht. Das erhaltene Phospholipidgemisch wurde unter Rühren und Inertgas in Wasser für Injektionszwecke ad 1000 ml dispergiert und danach mittels Hochdruckspalthomogenisator in fünf Umläufen auf einen mittleren Partikeldurchmesser von < 100 nm gebracht. Das entstandene Liposomensystem wurde anschließend unter sterilen Bedingungen über ein 0,2 »m Filter filtriert und unter Inertbegasung in 10,0 ml Ampullen abgefüllt. Das nach Beispiel 1 hergestellte Phosphatidylcholin/Soja-PG-Natrium Liposomensystem wies folgende Eigenschaften auf:

| | |
|---|---|
| Phospholipidgehalt: | 10% (m/V) |
| Aussehen: | transparente, leicht opalisierende Flüssigkeit |
| pH: | 6,1 |
| Viskosität: | 2,6 mPa.s |
| osmotischer Druck: | 0,49 (% NaCl) |
| Transmission (660 nm): | 75% |
| mittl. Partikeldurchmesser (Laserlichtstreuung): | 75 nm |
| Sterilität: | entspricht der Prüfung auf Sterilität, DAB 9 |
| Endotoxingehalt (Limulustest): | entspricht Anforderungen des DAB 9 |
| elektronenmikroskopische Charakterisierung (Kryofixierung): | 40-100nm unilamellare Liposomen, selten bilamellare Liposomen |

Aufgrund seiner Zusammensetzung kann dieses Produkt in folgenden Anwendungsgebieten verwendet werden: Atherosklerose, erhöhte Blutfettwerte, Hepatopathien jeder Genese.

### Beispiel 2

500 g Phospholipidmischung, bestehend aus 497,5 g Phosphatidylcholin hochrein, d.h. weniger als 10 Gew.% Verunreinigungen, und 2,5 g Soja-PG-Natriumsalz, hergestellt nach Beispiel 1, wurden in 6,5 l Wasser für Injektionszwecke unter Rühren und Inertgas dispergiert. Danach wurde mit Wasser für Injektionszwecke auf 8,0 l aufgefüllt. In einem separaten Ansatzgefäß wurden 2 kg Maltose in 1,5 l Wasser für Injektionszwecke unter Erwärmen auf 70° gelöst. Durch mehrere Umläufen in einem Hochdruckspalthomogenisator (APV Gaulin, Typ LAB 60) wurde das Phopholipidsystem auf einen mittleren Partikeldurchmesser von 56 nm gebracht, mit der Maltoselösung unter Rühren und Inertgas gemischt, mit Wasser für Injektionszwecke auf 10,0 l aufgefüllt, sterilfiltriert, unter aseptischen Bedingungen abgefüllt und gefriergetrocknet. Das nach der Gefriertrocknung entstandene Lyophilisat wies folgende Merkmale auf:

| | |
|---|---|
| Aussehen: | kristallines, schwach-gelbes Trockenpulver |
| Gehalt an Restfeuchte nach Karl Fischer: | < 0,7% |
| Gehalt an Phospholipiden: | 500 mg/Vial |
| Sterilität: | entspricht der Prüfung auf Sterilität nach DAB 9 |
| Endotoxingehalt (Limulustest): | entspricht den Anforderungen des DAB 9 |

Nach Redispergierung des Lyophilisates mit 8,3 ml Wasser für Injektionszwecke erhielt man ein Liposomensystem mit folgenden Eigenschaften:

| | |
|---|---|
| Aussehen: | transparente, leicht opalisierende Flüssigkeit |
| pH-Wert: | 6,5 |
| Viskosität: | 2,7 mPa.s |
| Transmission (660 nm): | 72% |
| mittl. Partikeldurchmesser (Laserlichtstreumethode): | 60 nm |

Das nach Beispiel 1 gefertigte Phospholipid-Liposomensystem und das nach Beispiel 2 gefertigte Lyophilisat kann für folgende Anwendungszwecke eingesetzt werden: Atherosklerose, erhöhte Blutfettwerte, Hepatopathien jeder Genese. Gegenüber des in Beispiel 1 gefertigten wäßrigen Liposomensystems hat das nach Beispiel 2 gefertigte Lyophilisat den Vorteil einer noch größeren Stabilität.

Die nach Beispiel 1 hergestellte Phospolipidmischung, bestehend aus Phosphatidylcholin und Soja-PG-Natriumsalz, kann sowohl zur Herstellung von unbeladenen, sterilfiltrierbaren Phosphatidylcholin-Liposomensystemen (Beispiel 1 + 2) als auch für die Herstellung von beladenen sterilen Liposomensystemen (Beispiel 3-5) verwendet werden.

### Beispiel 3

10 g des erfindungsgemäßen Phospholipidgemisches wurden entsprechend Beispiel 1 gemeinsam mit 0,1 g Propidiumjodid (DNA-Marker) in Ethanol gelöst und nach Trocknung unter Vakuum, Inertgas und Kühlung in 100 ml Wasser für Injektionszwecke dispergiert. Danach wurde ebenfalls unter Inertbegasung und Kühlung eine Ultraschallbehandlung durchgeführt bis ein mittlerer Partikeldurchmesser der Liposomen von 80 nm (Laserlichtstreuung) erreicht wird. Das Liposomensystem wurde danach über ein 0,2 »m Filter sterilfiltriert und unter Inertbegasung zur Hälfte in braune 1,0 ml Ampullen abgefüllt. In dem sterilen, mit Propidiumjodid beladenen Liposomensystem wurde mittels eines Dialyseverfahrens (Dianorm^{R}-Gerät, Zellulosetriacetatmembran NMGT 20000) der Anteil an liposomalgebundenem Propidiumjodid ermittelt. Danach betrug der liposomalgebundene Propidiumjodidanteil 29%. In der 2. sterilfiltrierten Hälfte wurde der Anteil nicht liposomalgebundenen Propidiumjodids mittels Ultrafiltration über eine Zellulosetriacetatmembran NMGT 20000 abgetrennt, die Liposomendispersion nochmals über 0,2 »m sterilfiltriert und zu 1,0 ml unter Inertbegasung in braune Ampullen abgefüllt. Die somit erhaltene Liposomendispersion wies folgende Eigenschaften auf:

| | |
|---|---|
| Phospholipidgehalt: | 100 mg/ml |
| Propidiumjodidgehalt: | 0,285 mg/ml |
| pH-Wert: | 7,2 |
| Viskosität: | 1,7 mPa.s |
| mittl. Partikeldurchmeser (Laserlichtstreuung): | 129 nm |

### Beispiel 4

18,4 g der in Beispiel 1 beschriebenen Phospholipidmischung wurden gemeinsam mit 0,92 g Chinolingelb in Ethanol gelöst, unter Vakuum zur Trockne gebracht, mit Wasser für Injektionszwecke ad 200 ml dispergiert und danach unter Kühlung ultraschallbehandelt. Das erhaltene Liposomensystem wurde anschließend sterilfiltriert und unter aseptischen Bedingungen in Injektionsflaschen zu 5,0 ml abgefüllt. Die sterilfiltrierte Liposomendispersion wies folgende Eigenschaften auf:

| | |
|---|---|
| Aussehen: | transparente, opalisierende gelbe Flüssigkeit |
| pH-Wert: | 6,4 |
| mittl. Partikeldurchmeser (Laserstreulichtmethode): | 75 nm |
| Transmission (660 nm): | 33 % |
| Sterilität: | entspricht der Prüfung auf Sterilität, DAB 9 |
| Chinolingelb, liposomalgebunden: | 1,38 mg/ml |
| Chinolingelb, nicht liposomalgebunden: | 3,2 mg/ml |

Zur Bestimmung des Anteils an liposomalgebundenem Chinolingelb wurde mittels Ultrafiltration über eine Zellulosetriacetatmembran NMGT 20000 der nicht liposomalgebundenen Chinolingelbanteil abgetrennt und photometrisch in der Liposomendispersion und im Filtrat der Anteil Chinolingelb bestimmt.

Die entsprechend Beispiel 2 in ein steriles Trockenpulver überführte erfindungsgemäße Phospholipidmischung eignet sich auch zur extemporierten (ready to use) Herstellung von mit aktiven wasserlöslichen Substanzen beladenen Liposomen.

### Beispiel 5

Steriles Trockenpulver, entsprechend 500 mg Phospholipidmischung beschrieben in Beispiel 1, und 2000 mg Trägerstoff wurden mit 5,0 ml Doxorubicin HCl-Lösung (10,0 mg Doxorubicin HCl) dispergiert. Das entstehende und mit Doxorubicin HCl beladene Liposomen-Redispergat (6,8 ml) wies einen Gehalt an Phospholipiden von 73,5 mg/ml und einen Gesamt-Doxorubicin HCl Gehalt von 0,735 mg/ml auf. Der Anteil an liposomal gebundenem Doxorubicin HCl wurde mit 0,58 mg/ml ermittelt und entspricht einer Einschlußrate von ca. 78%.

Die Ermittlung des liposomalgebundenen Doxorubicin HCl Anteils erfolgte nach der Dialysemethode mittels Liposomaten, Einsatz 5,0 ml, Dauer 5 Std.

### Beispiel 6

### Herstellung eines Leer-Liposomensystems

100 g Phosphatidylcholin hochrein, d.h. weniger als 10 Gew.% Verunreinigungen, und 0,502 g Soja-Phosphatidylglycerol-Natriumsalz (PG) werden in 500 ml Ethanol DAB 9 gelöst und anschließend unter Vakuum auf einen Trockensubstanzgehalt von 92 Gew.% eingestellt. Das erhaltene Phospholipidgemisch, bestehend aus 91,54 Gew.% Phosphatidylcholin, 0,46 Gew.% Soja-Phosphatidylglycerol-Natriumsalz, 6 Gew.% Ethanol und 2 Gew.% Wasser, wurde unter Rühren und Inertgas in Wasser für Injektionszwecke ad 1000 ml dispergiert und danach mittels Hochdruckspalthomogenisator in fünf Umläufen auf einen mittleren Partikeldurchmesser von < 100 nm gebracht. Das entstandene Liposomensysten wurde anschließend unter sterilen Bedingungen über ein 0,2 »m Filter filtriert und unter Inertbegasung in 10,0 ml Ampullen abgefüllt. Das nach Beispiel 6 hergestellte Phosphatidylcholin/Soja-PG-Natrium Liposomensystem wies folgende Eigenschaften auf:

| | |
|---|---|
| Phosphatidylcholingehalt: | 10% (m/V) |
| Aussehen: | transparente, leicht opalisierende Flüssigkeit |
| pH: | 6,1 |
| Viskosität: | 2,6 mPa.s |
| osmotischer Druck: | 0,49 (% NaCl) |
| Transmission (660 nm): | 75% |
| mittl. Partikeldurchmesser (Laserlichtstreuung): | 75 nm |
| Sterilität: | entspricht der Prüfung auf Sterilität, DAB 9 |
| Endotoxingehalt (Limulustest): | entspricht den Anforderungen des DAB 9 |

Aufgrund seiner Zusammensetzung kann dieses Produkt in folgenden Anwendungsgebieten verwendet werden: Atherosklerose, erhöhte Blutfettwerte, Hepatopathien jeder Genese.

### Beispiel 7

### Herstellung eines Leer-Liposomensystems

100 g Phosphatidylcholin hochrein, d.h. weniger als 10 Gew.% Verunreinigungen, und 0,502 g Soja-Phosphatidylglycerol-Natriumsalz (PG) werden in 500 ml Ethanol DAB 9 gelöst und anschließend unter Vakuum auf einen Trockensubstanzgehalt von 92 Gew.% eingestellt. Das erhaltene Phospholipidgemisch, bestehend aus 91,54 Gew.% Phosphatidylcholin, 0,46 Gew.% Soja-Phosphatidylglycerol-Natriumsalz, 6 Gew.% Ethanol und 2 Gew.% Wasser, wurde unter Rühren und Inertgas in Wasser für Injektionszwecke ad 8333 ml dispergiert und danach mittels Hochdruckspalthomogenisator bei 500 bar in fünf Umläufen auf einen mittleren Partikeldurchmesser von < 100 nm gebracht. Das entstandene Liposomensystem wurde anschließend unter sterilen Bedingungen über ein 0,2 »m Filter filtriert und unter Inertbegasung in 10,0 ml Ampullen abgefüllt. Dag nach Beispiel 7 hergestellte Phosphatidylcholin/Soja-PG-Natrium Liposomensystem wies folgende Eigenschaften auf:

| | |
|---|---|
| Phosphatidylcholingehalt: | 1,2% (m/V) |
| Aussehen: | transparente, leicht opalisierende Flüssigkeit |
| pH: | 6,19 |
| Viskosität: | 1,4 mPa.s |
| Transmission (660 nm): | 82% |
| mittl. Partikeldurchmesser (Laserlichtstreuung): | 58 nm |
| Sterilität: | entspricht der Prüfung auf Sterilität, DAB 9 |
| Endotoxingehalt (Limulustest): | entspricht den Anforderungen des DAB 9 |

## Patentansprüche

1. Wäßriges Liposomensystem, das ggf. ein nicht toxisches organisches Lösungsmittel, ein hochreines Phosphatidylcholin, mindestens einen phospholipidischen Ladungsträger sowie ggf. mindestens eine pharmazeutisch wirksame Substanz enthält, wobei das hochreine Phosphatidylcholin zu dem phospholipidischen Ladungsträger in einem Massenverhältnis von 50:1 bis 400:1, vorzugsweise von 100:1 bis 200:1, vorliegt, dadurch gekennzeichnet, daß das Liposomensystem aus dem hochreinen Phosphatidylcholin, dem mindestens einen phospholipidischen Ladungsträger, ggf. dem nicht toxischen organischen Lösungsmittel sowie ggf. der mindestens einen pharmazeutisch wirksamen Substanz und dem Wasser besteht, wobei das hochreine Phosphatidylcholin ein solches Phosphatidylcholin ist, das weniger als 10 Gew.% Verunreinigungen aufweist.

2. Liposomensystem nach Anspruch 1, dadurch gekennzeichnet, daß es als phospholipidischen Ladungsträger mindestens ein Salz, vorzugsweise ein Natrium- und/oder Ammoniumsalz, von Phosphatidylglycerol und/oder dessen Derivaten aufweist.

3. Liposomensystem nach Anspruch 2, dadurch gekennzeichnet, daß es als Ladungsträger ein Salz von Dimyristoylphosphatidylglycerol und/oder Dipalmitoylphosphatidylglycerol aufweist.

4. Liposomensystem nach Anspruch 2 oder 3, dadurch gekennzeichnet, daß das Phosphatidylglycerol ein Soja-Phosphatidylglycerol ist.

5. Liposomensystem nach einem der vorangehenden Ansprüche, dadurch gekennzeichnet, daß es zwischen 0,5 Gew.% und 20 Gew.% Phosphatidylcholin enthält.

6. Liposomensystem nach einem der vorangehenden Ansprüche, dadurch gekennzeichnet, daß es mindestens eine pharmazeutisch wirksame Substanz beinhaltet.

7. Liposomensystem nach Anspruch 6, dadurch gekennzeichnet, daß die wirksame Substanz ein Wirkstoff zur Behandlung von Krebs, Aids, Leber-, Viruserkrankungen oder Pneumocyctis carinii Pneumonie ist.

8. Verfahren zur Herstellung eines Liposomensystems nach einem der Ansprüche 1 bis 7, dadurch gekennzeichnet, daß man zunächst das hochreine Phosphatidylcholin, das weniger als 10 Gew.% Verunreinigungen aufweist, zusammen mit dem phospholipidischen Ladungsträger in einem Massenverhältnis von 50:1 bis 400:1, vorzugsweise von 100:1 bis 200:1, in einem organischen Lösungsmittel löst oder dispergiert, anschließend die Lösung bzw. die Dispersion einengt und hiernach Wasser unter Ausbildung des Liposomensystems zugibt.

9. Verfahren nach Anspruch 8, dadurch gekennzeichnet, daß man als organisches Lösungsmittel Ethanol, Propanol-1 und/oder Propanol-2 verwendet.

10. Verfahren nach Anspruch 8 oder 9, dadurch gekennzeichnet, daß man die Lösung bzw. Dispersion auf ein Restvolumen an Lösungsmittel von 0 Vol.% bis 30 Vol.%, vorzugsweise 5 Vol.% bis 10 Vol.%, einengt.

11. Verfahren nach einem der Ansprüche 8 bis 10, dadurch gekennzeichnet, daß man nach der Zugabe des Wassers das hierbei entstehende Liposomensystem einer Hochdruckspalthomogenisation oder einer Ultraschallbehandlung unterwirft.

12. Verfahren nach Anspruch 11, dadurch gekennzeichnet, daß man die Hochdruckspalthomogenisation bzw. die Ultraschallbehandlung so lange durchführt, bis die dabei gebildeten Liposome einen mittleren Durchmesser zwischen 50 nm und 180 nm aufweisen.

13. Verfahren nach einem der Ansprüche 8 bis 12, dadurch gekennzeichnet, daß man das Liposomensystem über einen 0,2 »m Filter filtriert.

14. Verfahren nach einem der Ansprüche 8 bis 13, dadurch gekennzeichnet, daß man das durch die Zugabe des Wassers gebildete Liposomensystem nach Zusatz eines geeigneten Hilfsstoffes, insbesondere eines Kohlehydrates, schonend trocknet, insbesondere gefriertrocknet.

15. Verfahren zur Herstellung eines mit einem Wirkstoff versehenen Liposomensystems nach einem der Ansprüche 6 bis 14, dadurch gekennzeichnet, daß man den Wirkstoff zusammen mit dem hochreinen Phosphatidylcholin und dem phospholipidischen Ladungsträger in dem organischen Lösungsmittel löst, emulgiert oder dispergiert.

16. Verfahren zur Herstellung eines mit einem Wirkstoff versehenen Liposomensystems nach Anspruch 15, dadurch gekennzeichnet, daß man das getrocknete Liposomensystem in Wasser, dem mindestens eine pharmazeutisch wirksame Substanz zugesetzt ist, aufnimmt.

## Claims

1. An aqueous liposome system, containing optionally a non-toxic organic solvent, a highly pure phosphatidylcholine, at least one phospholipidic charge carrier and optionally at least one pharmaceutically active substance, said highly pure phosphatidylcholine being present relative to said phospholipidic charge carrier in a mass ratio of 50 : 1 to 400 : 1, preferably of 100 : 1 to 200 : 1, characterized in that the liposome system consists of the highly pure phosphatidylcholine, the at least one phospholipidic charge carrier, optionally the non-toxic organic solvent and optionally the at least one pharmaceutically active ingredient and water, whereby said highly pure phosphatidylcholine is such a phosphatidylcholine, which contains less than 10 % by weight of impurities.

2. The liposome system according to claim 1, characterized in that it comprises as phospholipidic charge carrier at least one salt, preferably a sodium and/or ammonium salt, of phosphatidylglycerol and/or its derivatives.

3. The liposome system according to claim 2, characterized in that it comprises as phospholipidic charge carrier a salt of dimiristoylphosphatidylglycerol and/or dipalmitoylphosphatidylglycerol.

4. The liposome system according to claim 2 or 3, characterized in that the phosphatidylglycerol is a soybean phosphatidylglycerol.

5. The liposome system according to one of the preceding claims, characterized in that it contains between 0,5 % by weight and 20 % by weight phosphatidylcholine.

6. The liposome system according to one of the preceding claims, characterized in that it comprises at least one pharmaceutically active ingredient.

7. The liposome system according to claim 6, characterized in that the active ingredient is an active ingredient for the treatment of cancer, aids, liver diseases, virus diseases or Pneumocystis carinii pneumonia.

8. A method for the preparation of a liposome system according to one of the claims 1 to 7, characterized in that initially the highly pure phosphatidylcholine, comprising less than 10 % by weight of impurities, is dissolved or dispersed with the phospholipidic charge carrier in a mass ratio of 50 : 1 to 400 : 1, preferably of 100 : 1 to 200 : 1, in an organic solvent, subsequently the solution or the dispersion is concentrated and hereafter water is added, leading to the formation of the liposome system.

9. The method according to claim 8, characterized in that ethanol, 1-propanol and/or 2-propanol is used as organic solvent.

10. The method according to claim 8 or 9, characterized in that the solution or dispersion is concentrated to a residual volume of the solvent of 0 % by volume to 30 % by volume, preferably 5 % by volume to 10 % by volume.

11. The method according to one of the claims 8 to 10, characterized in that the liposome system resulting after the addition of water is subjected to a high pressure split homogenisation or an ultrasonic treatment.

12. The method according to claim 11, characterized in that the high pressure split homogenisation or the ultrasonic treatment is performed for such a time, until the resulting liposomes have a mean diameter between 50 nm and 180 nm.

13. The method according to one of the claims 8 to 12, characterized in that the liposome system is filtered through a 0,2 »m filter.

14. The method according to one of the claims 8 to 13, characterized in that the liposome system formed after the addition of water is gently dried, in particular lyophilised, after the addition of a suitable excipient, in particular a carbohydrate.

15. The method for the preparation of a liposome system containing an active ingredient according to one of the claims 6 to 14, characterized in that the active ingredient is dissolved, emulsified or dispersed with the highly pure phosphatidylcholine and the phospholipidic charge carrier in the organic solvent.

16. The method for the preparation of a liposome system containing an active ingredient according to claim 15, characterized in that the dried liposome system is taken up in water, to which the at least one pharmaceutically active ingredient has been added.

## Revendications

1. Système aqueux à liposomes, qui contient éventuellement un solvant organique atoxique, une phosphatidylcholine de haute pureté, au moins un porteur de charges phospholipidique, ainsi qu'éventuellement au moins une substance pharmaceutiquement active, où la phosphatidylcholine de haute pureté se présente, vis-à-vis du porteur de charges phospholipidique, en un rapport massique de 50:1 à 400:1, de préférence de 100:1 à 200:1, caractérisé en ce que le système à liposomes se compose de la phosphatidylcholine de haute pureté, de l'au moins un porteur de charges phospholipidique, éventuellement du solvant organique atoxique, comme éventuellement aussi de l'au moins une substance pharmaceutiquement active, où la phosphatidylcholine de haute pureté est une phosphatidylcholine du genre de celles qui présente moins de 10% en poids d'impuretés.

2. Système à liposomes suivant la revendication 1, caractérisé en ce qu'il présente, à titre de porteur de charges phospholipidique, au moins un sel, de préférence un sel de sodium et/ou d'ammonium, du phosphatidylglycérol et/ou de ses dérivés.

3. Système à liposomes suivant la revendication 2, caractérisé en ce qu'il présente, à titre de porteur de charges, un sel du dimyristoylphosphatidylglycérol et/ou du dipalmitoylphosphatidylglycérol.

4. Système à liposomes suivant la revendication 2 ou 3, caractérisé en ce que le phosphatidylglycérol est un phosphatidylglycérol de soja.

5. Système à liposomes suivant l'une quelconque des revendications précédentes, caractérisé en ce qu'il contient entre 0,5% en poids et 20% en poids de phosphatidylcholine.

6. Système à liposomes suivant l'une quelconque des revendications précédentes, caractérisé en ce qu'il contient au moins une substance pharmaceutiquement active.

7. Système à liposomes suivant la revendication 6, caractérisé en ce que la substance active est un principe actif pour le traitement du cancer, du sida, de maladies du foie, de maladies virales ou de Pneumocyctis carinii Pneumonie.

8. Procédé de préparation d'un système à liposomes suivant l'une quelconque des revendications 1 à 7, caractérisé en ce que l'on dissout ou disperse d'abord la phosphatidylcholine de haute pureté, qui présente moins de 10% en poids d'impuretés, en même temps que le porteur de charges phospholipidique dans le rapport massique de 50:1 à 400:1, de préférence, de 100:1 à 200:1, dans un solvant organique, on concentre ensuite la solution ou la dispersion et on ajoute alors de l'eau en vue de la formation du système à liposomes.

9. Procédé suivant la revendication 8, caractérisé en ce que l'on utilise, à titre de solvant organique, de l'éthanol, du propanol-1 et/ou du propanol-2.

10. Procédé suivant la revendication 8 ou 9, caractérisé en ce que l'on concentre la solution à la dispersion jusqu'à un volume résiduel en solvant de 0% en volume à 30% en volume, de préférence de 5% en volume à 10% en volume.

11. Procédé suivant l'une quelconque des revendications 8 à 10, caractérisé en ce qu'après l'addition de l'eau, on soumet le système à liposomes à une homogénéisation à scission sous haute pression ou à un traitement par des ultrasons.

12. Procédé suivant la revendication 11, caractérisé en ce que l'on entreprend l'homogénéisation à scission sous haute pression ou le traitement par des ultrasons pendant la durée nécessaire pour que les liposomes ainsi formés présentent un diamètre moyen compris entre 50 nm et 180 nm.

13. Procédé suivant l'une quelconque des revendications 8 à 12, caractérisé en ce que l'on filtre le système à liposomes à travers un filtre de 0,2 »m.

14. Procédé suivant l'une quelconque des revendications 8 à 13, caractérisé en ce que l'on sèche de manière ménagée, plus spécialement lyophilise ou sèche par congélation, le système à liposomes formé par l'addition d'eau après addition d'un adjuvant convenable.

15. Procédé de préparation d'un système à liposomes pourvu d'un principe actif, suivant l'une quelconque des revendications 6 à 14, caractérisé en ce que l'on dissout, émulsionne ou disperse le principe actif en même temps que la phosphatidylcholine de haute pureté et le porteur de charges phospholipidique dans le solvant organique.

16. Procédé de préparation d'un système à liposomes pourvu d'un principe actif, suivant la revendication 15, caractérisé en ce que l'on reprend dans de l'eau, le système à liposomes séché auquel on ajoute au moins une substance pharmaceutiquement active.
